# EUROPEAN PATENT APPLICATION

(11) **EP 4 524 554 A1**
(43) Date of publication of application: **19.03.2025**
(21) Application number: 23461651.4
(22) Date of filing: 18.09.2023
(51) Int. Cl.: G01N 22/00, H01P 7/06, G01N 33/24

(54) **ELECTROMAGNETIC RESONATING STRUCTURE AND A METHOD OF MEASURING A MATERIAL PARAMETER**

(71) Applicant: Qwed Spolka Z Ograniczona Odpowiedzialnoscia, 02-078 Warszawa (PL)
(72) Inventor: GWAREK, Wojciech, 02-078 Warszawa (PL)
(74) Representative: Bury & Bury

(57) **Abstract**

Electromagnetic resonating structure **(100, 200,300,400)** comprising a substantially axially symmetrical cavity **(101, 102, 401)** having an axis of symmetry **(Z)** and at least partially surrounded by a wall **(111, 112, 411)** made of conducting material and adapted to support electromagnetic resonance at working mode having axially symmetrical all field components and E-field restricted to angular direction, according to the invention is provided with at least one channel **(120, 220, 320, 420)** extending outwards from the cavity **(101, 102, 401)** and connected with a volume of absorbing material **(130, 330, 430),** wherein the channel **(120, 220, 320, 420)** is configured to receive a field distributed according to at least one mode other than working mode and reflect back to the cavity field distributed according to the working mode.

A method of measuring parameter of a material according to the invention involves comparison of measurements done using an empty resonating structure according to the invention and resonating structure according to the invention with a sample under test inside.

## Description

### Field of the invention

The invention concerns electromagnetic resonating structure especially for measurement purposes and a method of measuring of the material parameter or parameters using a resonator. Typically what is measured is permittivity and loss factor of the material.

### State of the art

Electromagnetic cavity resonating structures, so called resonators, are widely used in material measurements for a wide range of applications including electronics, telecommunications and space industry.

Electromagnetic field in the resonator can have only certain patterns, so called modes. Available modes depend on the shape of the cavity and frequency of the induced field.

Axially symmetrical electromagnetic cavity resonators are relatively easy to manufacture and hence popular in the art. The modes available therein are generally divided into two types TE (transverse electric) modes and TM (transverse magnetic modes) . Specific TE mode with indices m (azimuthal), n (radial), and p (longitudinal) is commonly written as TEmnp.

The cavity resonators are multi-resonant structures and often resonance at the same frequency can be obtained in more than one mode. That involves a risk that measurement performed with desired (measurement) mode is disadvantageously affected by other parasite mode, or modes. The mode which is intended to be used in the resonator is often referred to as desired, measurement or working mode.

Modes TEOnp, especially TE011 are particularly well suited for measurements in various configurations. Fig. 1 and Fig. 2 show magnetic field distribution of TE011 mode while Fig. 3 shows a vertical cross-section of the axially symmetrical cavity resonator with indication of currents flowing in the cavity walls. Notably all TEOnp modes have currents flowing in direction perpendicular to a plane of vertical cross-section.

In the resonators partly filled with dielectrics the field distribution may divert from classical TEOnp types but still have distributions of all field components symmetrical. That kind of modes are often referred to as TE0nδ.

US20210063332 discloses an example of a split cylinder resonator (SCR) that is a resonator having a wall divided in two parts by a plane perpendicular to the axis of symmetry. Specifically, the resonator of US20210063332 has: a first conductive body having a first cavity formed in a cylindrical shape having the side surface and the bottom surface, a second conductive body having a second cavity formed in a cylindrical shape having the side surface and the bottom surface and arranged so that the second cavity faces the first cavity. The electromagnetic field is induced with first and second coaxial cables respectively having first and second loop antennas at a tip, the first and second loop antennas being exposed to an integrated cavity which is formed by the first cavity and the second cavity, the first and second coaxial cables facing each other. Nevertheless, multiple other methods of coupling the resonators are known in the art. The resonator of US20210063332 is used with TE011 because of its large Q-factor of the resonance and distribution of electric field allowing uniform application to the measured sample. US20210063332 mentions known problem with TE011 mode, which is difficulty in separation from other modes, especially TM111, as the ranges of allowable mode frequencies overlap. Accordingly, any perturbance inside the cavity (e.g. measured sample) results in inducing additional, unwanted modes and effectively the measurement result becomes ambiguous or inaccurate. To solve this problem US20210063332 offers alteration in the shape of the known cavity and particular way of coupling. Similar disclosure concerning split cylinder resonator can be found in JP2006300856.

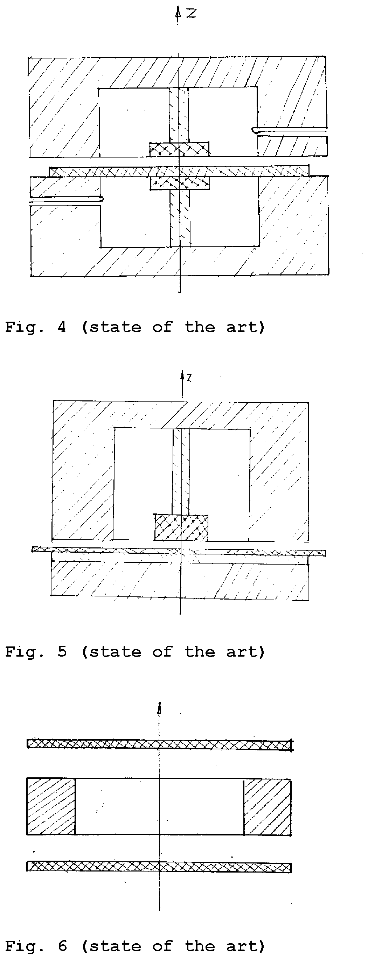

In the art there are also known other methods using resonators with cavities being a body of revolution (axially symmetrical). These methods include Split Post Dielectric Resonator (SPDR), SiPDR (Single Post Dielectric Resonator). Example of SPDR resonator is shown in Fig. 4. It is used for measurements of low-loss dielectric material. An example of SiPDR resonator is shown in Fig. 5 it is used predominantly in measurements of surface resistances e.g in susceptors for microwave food packages.

Fig. 6 shows schematically another known solution - a simple cylindrical cavity resonator with one or both bottoms removable and replaceable by measured metal surface. When the cylinder is of relatively large diameter and low height, most of the metal losses are in the bottoms, which makes the Q-factor results sensitive mostly to the measured metal surfaces.

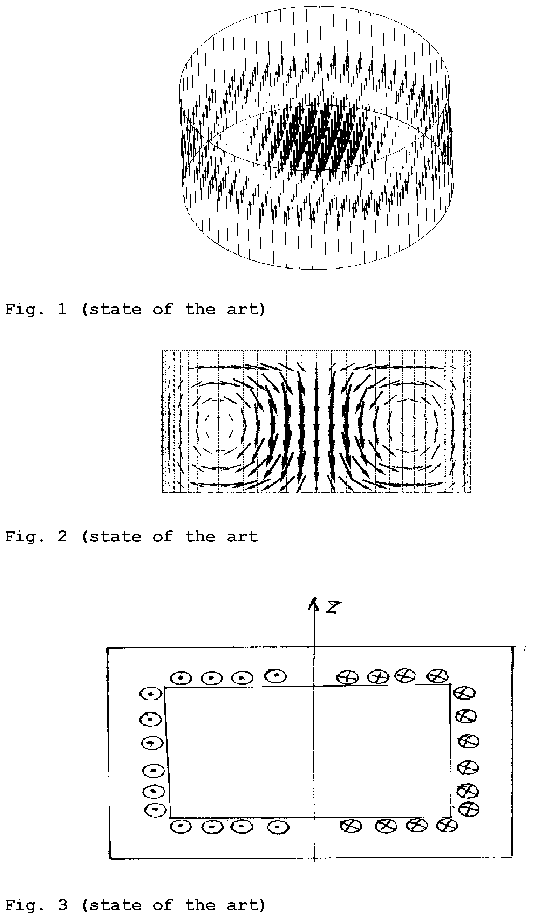

All the structures presented in Figs. 1-7 are cavity resonators in which cavities have axial symmetry and therefore can be modelled as bodies of revolution. All inner parts of the cavity (filled with air or dielectric materials) are axially symmetrical with respect to a vertical axis. The only components not meeting this criterion are coupling elements. However, naming convention in the field omits said element but rather refer to the general shape of cavity. Coupling elements need to be small enough not to perturb decisively the field distributions of the natural (axisymmetrical) eigenmodes of the cavity.

The measured samples being inserted between the resonator halves or at the cavity bottoms, do not need to be bodies of revolution providing that their lateral size is bigger than the cavity diameter and they are centrally placed to cover the entire lateral size of the cavity.

In all cases discussed here we will be applying the modes of the TEOnp class. These modes allow configuring the cavity for opening as they are not susceptible to cuts in the walls perpendicular to axis of symmetry Z or leaving one of the bases open for attaching the sample. Beside the positive features mentioned above, the modes TEOnp have a feature extremely important for measurement of material properties of flat samples of materials. When that mode is applied, the material properties are measured using electric field components tangential to the sample. It is well known that the measurement with field of such a direction assures the best accuracy since it makes the measurement results practically insensitive to thin air slots between the measured sample and the test fixture. Such slots typically ruin the accuracy of measurements when the E-field is normal to the measured material surface, especially when we measure a sample of relatively small thickness and high permittivity. Beside the advantages presented above, application of TEOnp modes has one clear disadvantage. Even mode TE011, which has the lowest resonant frequency in that group, is never the dominant mode of all modes excited in the cavity. Since it is difficult to control all the other excited modes, the resonance of the desired mode is typically hidden in a group of other modes - discussion of modes in the resonators can be found under an address https://www.qwed.eu/resonators.html. Consequently, all mentioned resonators suffer from the problem of inducing additional modes and reduction of Q-factor or introducing a measurement ambiguity as a consequence thereof. Those additional modes are usually referred to as unwanted or parasitic.

The problem has been mentioned in a Technical Overview of 85072A 10 GHZ Split Cylinder Resonator by Keysight Technologies (https://www.keysight.com/us/en/assets/7018-01496/technical-overviews/5989-6182.pdf) mentioning that mere presence of a sample may cause shifting the useful mode resonant frequency down and consequently interference with non TE modes. The only recommendation given to mitigate this is changing thickness of the sample which is not always easy, and in case of many applications, e.g. wafers for integrated circuits - not always possible. Generally measurements of thicker samples are more susceptible to contamination with higher order modes.

### Problem to be solved

It is an object of the present invention to solve a problem of contamination of working mode TEOnp with different (parasitic) modes in measurements with axially symmetrical cavity resonators which is particularly difficult to alleviate with samples having larger thickness.

### Summary of the invention

The invention concerns electromagnetic resonating structure comprising a substantially axially symmetrical cavity having an axis of symmetry and at least partially surrounded by a wall made of conducting material and adapted to support electromagnetic resonance at working mode having axially symmetrical all field components and E-field restricted to angular direction traditionally referred to as TEOnp or TE0nδ. According to the invention the resonating structure is provided with at least one channel extending outwards from the cavity and connected with a volume of absorbing material, wherein the channel is configured to receive a field distributed according to at least one mode other than the TEOnp or TE0nδ and reflect back to the cavity field distributed according to the working mode. Modes TEOnp and TE0nδ are not affected due to particular distribution of current in the resonator walls and they do not interact with the absorbing material. Parasitic mode does interact with the absorbing material and hence is dumped. Consequently impact of parasitic modes on the measurement is mitigated.

Advantageously the channel has a height within a range of 0.02 λ to 0.1 λ.

Advantageously the channel has a length within a range of 0.2 λ to 0.7 λ.

Advantageously the working mode is TE011.

The electromagnetic resonating structure advantageously has more than one channel. This enhances the dumping of parasitic modes.

The electromagnetic resonating structure according to any of claims advantageously has at least one channel extending in a direction perpendicular to the axis of symmetry of the cavity.

The electromagnetic resonating structure advantageously has a wall divided into a first wall defining a first part of the cavity and a second wall defining a second part of the cavity. The structure is provided with at least two channels each located in different part of the cavity. This makes the damping effect less dependent on a tolerance of division of the wall.

The electromagnetic resonating structure advantageously has at least one channel extending in parallel to the axis of symmetry of the cavity. Channels extending in parallel and perpendicular to the axis of symmetry of the cavity can be combined however it leads to increase in size of the structure.

The conducting wall advantageously is divided into two parts by a plane perpendicular to the axis of symmetry of the cavity leaving a slot adapted to receive a flat dielectric sample. Thus sample introduces least disturbance. During measurement the sample is inserted into the slot between the wall parts providing a possibility to determine properties of that sample by change of the resonant frequency and Q-factor of the structure.

Alternatively the wall has an open base perpendicular to the axis of symmetry of the cavity and the open base is adapted to be applied to a flat sample under test. This is dedicated to measurement of the samples provided on metallic surfaces or conductivity of the flat metallic surfaces. In the latter case the conductivity of the sample can be determined by the change of the Q-factor of the resonating structure, as described in the paper: Krupka, J. Contactless methods of conductivity and sheet resistance measurement for semiconductors, conductors and superconductors. Meas. Sci. Technol. 24 (2013) 062001.doi:10.1088/0957-0233/24/6/062001.

The electromagnetic resonating structure advantageously has at least one axially symmetrical dielectric insert with the axis of symmetry coinciding with that of the cavity, adapted for supporting a sample.

Preferably the structure comprises two dielectric inserts arranged symmetrically to the axis of symmetry of the cavity and is adapted for sandwiching the sample between them.

The insert preferably comprises sapphire.

Advantageously the cavity is divided into a first part and second part and wherein each of the first part and the second part is closed with one of the two dielectric inserts located in a slot next to a cut separating the first part and the second part. The wall of the cavity comprises additional channel beginning next to the slot and comprising absorbing material at its end. This additional channel allows damping parasitic modes induced by the dielectric insert.

The invention further concerns a method of measuring a material parameter of a sample under test, using a resonating structure comprising cavity. The method comprises steps of inducing a working resonant mode of electromagnetic field, in the resonating structure without the measurement sample and measuring a first response. Then the sample under test is placed in the resonating structure, the same working resonant mode of radiation is induced in the resonating structure a second response is measured. Then the material parameter of the sample is determined from the difference between the first and the second response. The resonating structure is the resonating structure according to the invention. The working mode is TEOnp or TE0nδ.

The invention is based on an observation that the advantages of the TEOnp modes in so many applications are all based on the fact, that those modes produce no vertical currents at the cylinder walls and no radial currents at the cylinder bottoms as shown in Fig 3. All the other modes do produce such currents and therefore enter channels and are subjected to the attenuation in the absorbing material. Accordingly, the configuration of channel and absorbing material works as choke for all modes except of useful ones. As a result, application of the invention removes most of restrictions concerning applicability of the resonators to samples of specific thickness.

This configuration has been named Q-Choke by the inventor. A resonating structure including a Q-Choke has been named a "Q-Choked resonator".

### Description of drawings

The invention has been described below in detail, with reference to the following figures.
Fig. 1 shows H-field distribution in a known cylindrical resonator with TE011 mode in perspective view;
Fig. 2 shows distribution of Fig. 1 in a vertical section;
Fig. 3 shows distribution of current in the wall of the cavity resonator known in the art;
Fig. 4 shows an example of SPDR resonator;
Fig. 5 shows an example of SiPDR resonator;
Fig. 6 shows an example of resonating structure with open bases

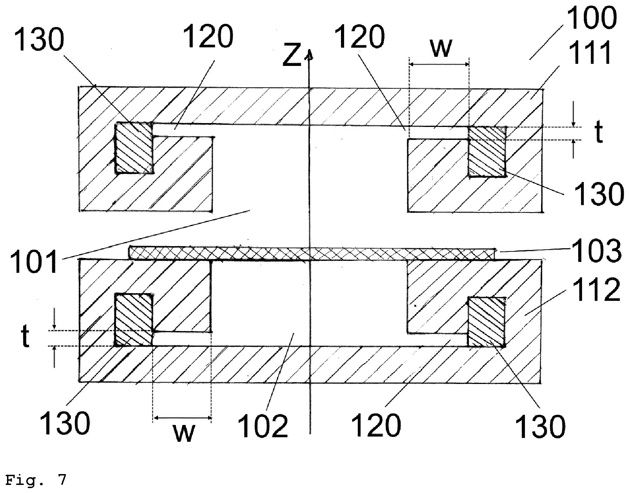

Fig. 7 shows schematically a vertical cross-sectional view of an embodiment of the resonating structure according to the invention having channels arranged perpendicularly to axis of symmetry Z;

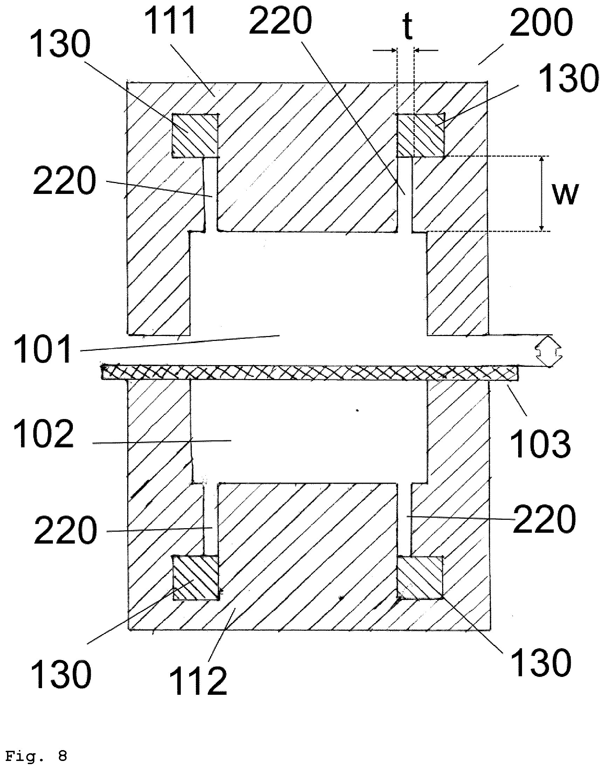

Fig. 8 shows schematically a vertical cross-sectional view of an embodiment of the resonating structure according to the invention having channels arranged in parallel to axis of symmetry Z;

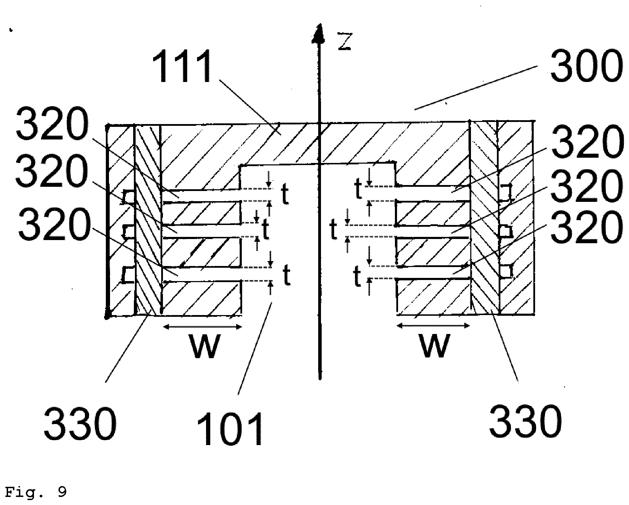

Fig. 9 shows schematically a vertical cross-sectional view of an embodiment of an upper part of the resonating structure according to the invention having multiple channels arranged in parallel to axis of symmetry Z and connected to vertical openings filled with attenuating materials;

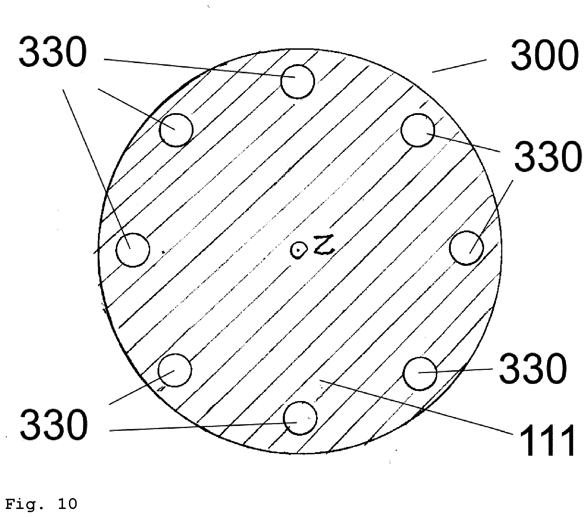

Fig. 10 shows schematically a top view of the embodiment of Fig. 9;

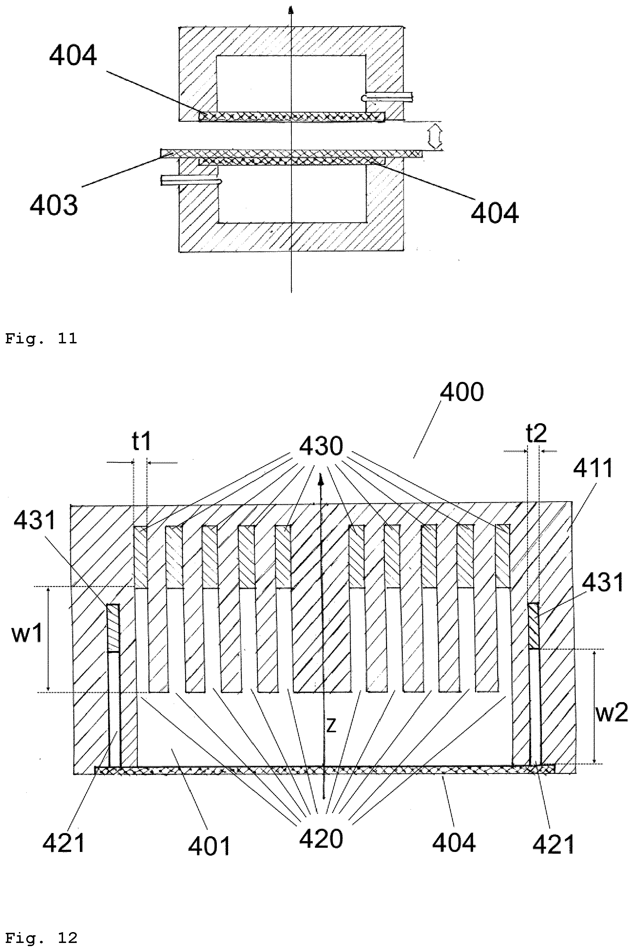

Fig. 11 shows schematically a configuration of resonator with additional dielectric plates sandwiching the sample(channels and pockets not shown).
Fig. 12 shows schematically a vertical cross-sectional view of an embodiment of the invention in configuration shown in Fig. 11 - top half without a sample under test.

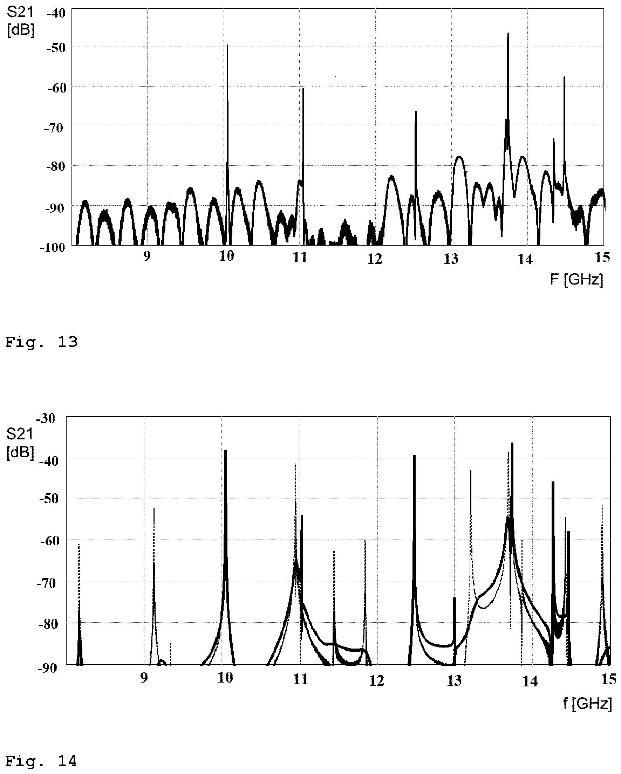

Fig. 13 shows measurement results of the embodiment shown in Fig. 7.
Fig. 14 shows simulation comparison of the same structure with and without use of the invention.

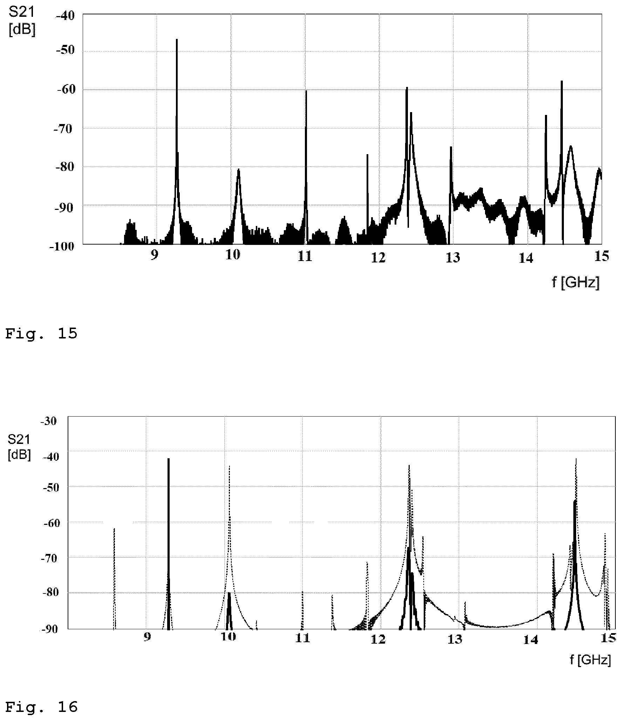

Fig. 15 shows measurement results of the embodiment shown in Fig. 7 comprising a sample under test.
Fig. 16 shows simulation comparison of the same structure comprising the same sample with and without use of the invention.

The first embodiment is described below with reference to Fig. 7 which shows vertical cross-section of a resonating structure **100** according to the invention. This embodiment is arranged in SCR configuration. The cavity is a cylinder having a vertical axis of symmetry **Z.** Wall of the resonating structure **100** comprises a first part **111** of a wall, and a second part **112** of a wall. Both parts **111, 112** of the wall are made of conducting material. The first and the second parts **111, 112** of the wall are split along plane perpendicular to the axis of symmetry **Z** and are adapted to be detached from each other. When stacked together parts **111, 112** form a wall surrounding the cavity of resonating structure. The cavity comprises two parts. A first part **101** of the cavity is surrounded by the first part **111** of the wall. A second part **102** of the cavity is surrounded by the second part **112** of the wall. For measurements a sample under test **103** is located between the walls **111, 112** in a manner known in the art for SCR resonators. Desired working mode for the measurement is TE011, but also higher order TEOnp modes can be used.

When the sample is flat and thinner than 0.3 wavelength in the measured sample the slot between the parts of the wall is reflective for TEOnp modes.

The structure **100** is provided with channels **120** extending outwards from the cavity. The channels do not need to be strictly symmetrical with the axis of rotation Z along all their length, providing that the non-symmetrical parts are sufficiently distant from the cavity so as not to deteriorate the ability of the slot to reflect TEOnp modes. Usually, 0.7 λ is sufficiently far but often 0.1 λ is enough. They do not constitute a part of the cavity as they are arranged so that they are negligible for TE011 mode.

The channels **120** are connected to pouches of absorbing material **130** adding such arrangement to axially symmetrical cavity resonator with conducting wall results in damping parasitic modes. This structure is referred to by inventor as Q-Choke.

Channels **120** are adjacent to the cavity resonator and have a form of a ring with axial dimension t much smaller than the wavelength λ at measurement frequency.

In the discussed embodiment both channels have height **t** of 0.05 λ. However it has been tested that very good measurement results are obtained when channels extending horizontally have a height **t** within a range of 0.02 λ < t < 0.1 λ. Length **w** of channel should be comparable to the wavelength to guarantee no interaction of the desired working mode TEOnp with absorbing material. In a present embodiment length of both channels **120** was equal to 0.5 λ. Good results are obtained for lengths within a range of 0.2 λ < t < 0.7 λ.

In the present embodiments channels **120** are realized as hollow space in a low-loss conductor material, filled with air. For reduction of dimension a low-loss dielectric filing can also be applied. Generally channels **120** can have arbitrary shapes but in practice toroid generated with rectangular surface **w×t** is easy to manufacture and to model in simulations.

One side of each channel, the input, is adjacent to the cavity of the resonator and perforating its wall. Thus, the magnetic field of the desired TEOnp mode is directed along its shorter dimension **t.** The surface currents excited by that mode in the resonator wall are in angular direction (perpendicular to the surface of Fig.7). They are evanescent along the longer dimension **w**, and there is no electric field along dimension ***t*.** Distribution of surface currents for exemplary TE011 mode is shown in Fig. 3. Distribution of currents causes the desired working mode to be totally reflected from the channel input and restricted from propagating in it.

TEOnp modes produce no vertical currents in the wall of the axially symmetrical cavity and no radial currents in the bottoms - as shown in Fig 2. All the other modes do produce such currents. Those vertical currents in the side of the wall and radial currents in the bottom of the wall, are diverted into channels **120** guiding radiation toward the absorbing material **130.** Consequently, the energy of unwanted parasitic modes is dissipated in the absorbing material **130** but energy of the desired measurement mode remains unaffected.

The inputs of channels **120** are reflective to the desired measurement TEOnp modes and hence there is no deterioration of the properties of the desired modes.

The parasitic modes are damped while desired modes are unaffected. SCR according to this embodiment allows more accurate and unambiguous measurements of a wide range of samples not measurable in conventional SCR.

Although SCR is given as example, analogous configuration can be provided for other known cavity resonators having substantially axially symmetrical cavities.

Usually it is enough to damp just one mode that can be induced at a frequency close to the frequency of the measurement one.

Although in the embodiment shown in Fig. 7 two channels 120 are provided, significant improvement can be obtained even with one channel.

Q-Chokes are applicable to axisymmetric microwave cavity resonators to dump the modes other than the modes of the TEOnp group. Often it is reasonable to apply more than one Q-Chokes applied to a resonator.

Another embodiment of the invention is shown in Fig. 8. It concerns SCR resonator with the same parts, but in this embodiment the channels **220** extend vertically outwards from the cavity and lead to pouches of absorbing material **130.** The lower dimension t of the channels **220** is horizontal in this embodiments, while the larger - the length **w** is vertical. This configuration provides analogues effect as the one discussed with respect to the previous embodiments. It is because the currents in top and bottom parts of the wall for mode TEOnp have no radial component, only angular. Therefore, any narrow vertical cut does not perturb the field distribution of the TEOnp mode in the cavity.

Channels **220** extend in whole angular range. In this embodiment a channel **220** has a width **t** is equal 0.05 λ and the channel length **w** is 0.3 λ.

It is also possible to use more channels and provide common pouches of absorbing material. Such embodiment of the invention, with horizontal channels extending radially is shown in Fig. 9 and Fig. 10. Fig. 9 shows vertical cross-section of a first part **111** of a wall of SCR resonator and Fig. 10 shows top view thereof. The first part **111** of a wall defining upper part **101** of the cavity is provided with three channels **320** in a form of symmetrical corrugations extending in whole range of angles and having width **t** equal to 0.1 λ and length **t** of 0.7 λ. Pouches for absorbing material **330** are realized as eight vertical openings extending in parallel to **Z** axis distributed angularly. Accordingly, every pouch intersects with multiple channels **320** at given length.

Another example of use of axially symmetrical cavity for measurements is a SCR with two additional dielectric plates **404** used for "sandwitching" a sample under test, shown in Fig. 11. This embodiment helps to concentrate the electric field in the sample **403** and the plates **404,** what limits the amplitude of magnetic field close to metal walls and consequently increases the Q-factor of the empty resonator. Preferred material for such plates is sapphire. It makes the process of measurement easier and can prevent contamination of the cavity, which is split into two cavities, each closed with dielectric plate - see Fig. 11. Unfortunately using such dielectric inserts increases a risk of inducing parasitic modes.

In the embodiment discussed with reference to Fig. 11 and Fig. 12 Q-Chokes were applied to a resonating structure **400.** Fig. 12 shows top half of the resonator, having a part **411** of the resonator wall defining part **401** of the cavity closed with sapphire plate **404.** The sapphire plate **404** has diameter 38 mm and thickness 0.4 mm.

There are five vertical Q-Chokes having inputs in a base of the cavity. They comprise channels **420** provided in a form of corrugations having width **t1.** These corrugations are air-filled along length **w1** in their parts close to the cavity and filled with an absorbing material **430** in their deepest parts as shown in Fig.12. Simulations and experiments have shown that good results can be obtained by using for that purpose rings 3-D printed from conductive PLA material. That type of material, while showing relatively good conductivity at low frequencies works well as absorbing material at microwave frequencies. An advantage of PLA is that it is 3D printable. PLA conductive material is one typical 3-D printer filament charged heavily with graphene particles. Its DC conductivity was measured to be about 3 [S/m]. It is a typical value for plastic materials heavily doped with carbon particles. Properties of the Q-Choke are not particularly sensitive to the value of the conductivity and practically good results can be obtained within conductivity range of one order of magnitude. The structure like that of Fig. 12 can resonate at several regularly spaced TE0n1 modes opening the possibility of multi resonant measurements.

The bottom part (not shown in the figures) of the resonating structure **400** has a mirrored shape with regard to the upper part shown in Fig. 12. Thus, the wall surrounding the cavity is cut in half. The cut is provided with a slot for receiving the sapphire insert **404** so that when two parts of the cavity are stacked together without sample between them, the two parts of the surrounding wall touch each other despite two sapphire plates in the middle. In an example shown in Fig. 12 the outer plane of the sapphire plate **404** is exactly in the plane of cut ending the first part **411** of the wall.

Optionally the sapphire plate is provided with additional Q-Choke defined by channel **421** extending inside a wall **411** from the split in the resonating structure and having an input covered with plate **404.** The channel has width **t2.** Along the length **w2** the channel is air-filled and its ending is filled with absorbing material **431.** This additional Q-Choke is dedicated to attenuate parasitic modes, caused by presence of dielectric plate. It additionally reflects the desired TEOnp modes back to cavity making them better confined to it while absorbing the undesired modes. Both effects lead to lower leakage of the energy to the environment, helping to obey the EMI standards. Dimensions **w2** and **t2** may differ from **w1** and **t1** due to constructional reasons but electromagnetically the work similarly and work well in similar ranges.

A variant of the configuration shown in Fig. 7 has been experimentally tested. It is an SCR with horizontal cut basically as presented in Fig.7. It has been designed for a nominal resonant frequency of 10 GHz. The SCR cavity **101**, **102** has the diameter D=37.7 mm and the total height H=57.3 mm of combined first part **101** of the cavity and the second part **102** of the cavity. At the top and bottom of the cavity there are provided channels **120** of height t=2 mm and length w=6.15 mm - in a whole range of angles, symmetrically. At the end of the each of the channels **120** there are installed rings of the absorbing material **130** - conductive PLA of the height Z2-Z1 = 18.65 mm and the thickness R2-R1 = 6.25 mm.

In the first part **101** of the cavity an input antenna has been provided in a form of a horizontal loop so that it excites vertical (Hz) field close to the cylinder wall.

In the opposite, an output antenna is located in the second part **102** of the cavity. Accordingly, antenna is placed on the opposite side of the central plane and is rotated by 135° around the cylinder axis. Use of angle 135° instead of typical 180° allows easier elimination of certain unwanted modes.

Notably the antennas are not shown in Fig. 7. The antennas are not axially symmetrical with respect to axis **Z** - they are examples of tolerable exceptions to symmetry of the structure.

During measurements, the input and output antennas were connected to the output and input of a Vector Network Analyzer (VNA) so that transmission (versus frequency) through the resonator can be measured. The measurements are taken with an empty resonator and with the same resonator loaded with the measured sample placed between the resonator halves.

Fig.13 presents results of measurements of the transmission (|S21|[dB]) through the empty prototype of SCR10 manufactured by QWED, equipped with horizontal Q-Chokes. It is noted that there are visible resonances at f₀ ≃ 10.047 GHz, at f₁ ≃ 11.021 GHz, f₂ ≃ 12.476 GHz, f₃ ≃ 13.743 GHz, f₄ ≃ 14.263 and f₅ ≃ 14.469 GHz. It should be noticed that there are no parasitic resonances below 10 GHz - transmission is attenuated to c.a. -90 dB.

Fig.14 presents electromagnetic simulations (QuickWave 3D) of the same resonator - thick continuous lines. The same resonances can be identified at f₀ ≃ 10.050 GHz, at f₁ ≃ 11.045 GHz, f₂ ≃ 12.529 GHz, f₃ ≃ 13.747 GHz and f₄ ≃ 14.352 GHz and f₅ ≃ 14.491 GHz. There are no parasitic resonances below 10 GHz. The difference between simulated and measured resonant frequencies is in most cases lower than 0.5% confirming high accuracy of simulations.

Fig. 14 presents also simulations of the same resonating structure but without channels and absorbers according to the invention - dotted line. Notably the S21 curves in a neighborhood of the first resonance at f₀ ≃ 10.05 GHz with and without invention are the same. However, in the resonating structure without the invention there are two visible resonances of parasitic modes: at. ~8.15 GHz and at ~9.1 GHz.

Even though the resonance f₀ ≃ 10.05 GHz of the desired mode of an empty resonator is far away from the closest resonance of the parasitic mode ~9.1 GHz interference is still a risk in a resonator with sample under test. It is quite clear, that the applied Q-Chokes dump all the unwanted modes by at least 30 dB while not affecting the desired mode TE011.

Measured Q-factor for that mode was 24 800.

Fig. 15 shows measurements for the same resonator as in Fig. 13, but comprising a 0.4 mm high plate - a sample of Sapphire.

A method of measuring parameter of a material according to the invention is particularly suited for measurements of permittivity and loss factor. In the present embodiment the method involves uses of resonating structure according to the invention. Referring to use with an embodiment shown in Fig. 7, the method first comprises steps inducing a mode T011 of electromagnetic field having axially symmetrical all field components in the empty resonator and measuring a first (reference) response including the resonant frequency and Q-factor.

Then a sample of material is placed in the resonator and measurement is repeated to obtain the second (measurement) response.

A permittivity of the sample is determined from the difference between the reference response and the measured response. That is a standard procedure described for the SPDRs in detail in the IEC norm: IEC 61189-2-721:2015. IEC Webstore. https://webstore.iec.ch/publication/22343 while in the case of SCRs it is described in: https://www.ipc.org/sites/default/files/test methods docs/2-5-5-13.pdf. For other types of resonators, the procedure is analogous. Scientific background of the procedure is described in: Krupka, J. Microwave Measurements of Electromagnetic Properties of Materials. Materials 2021, 14, 5097. https://doi.org/10.3390/ma14175097.

The relation between the measured quantities (resonant frequency and Q-factor) and the material parameters is determined by electromagnetic modeling. In the simplest cases involving a purely cylindrical resonator, analytical methods can be used. In more complicated cases, like the resonators with application of the present invention, numerical methods of electromagnetic simulations need to be applied. There is a plethora of commercially available electromagnetic simulators, which can be applied for this purpose. A person skilled in the art is capable of selecting and applying adequate simulator. For preparation of this invention the inventor has applied QuickWave 3D (QW-3D) Simulator available from QWED.

Fig.16 shows comparison of electromagnetic simulations with use of the invention (solid line) and without it (dotted line) of the same resonator filed with sapphire sample. It can be seen that high purity of the desired resonant modes is maintained which decisively contributes to the measurement accuracy and unambiguity.

Concluding measurement and simulation experiments it should be stressed that:
A) SCR is a multi-resonant structure,
B) In the empty resonator of the considered type, the desired mode TE011 resonates slightly above 10 GHz. In the classical version, not equipped with structure of the present invention, there are many other unwanted modes which may be called as "parasitic". Several of them may be transmitted through the resonator at higher level than the desired one. Some have resonant frequency higher and some lower than the desired mode.
C) The situation becomes more complicated with the sample inserted into the resonator. The resonances may be reshuffled due to different coupling of the inserted sample to different modes. The desired resonance becomes very difficult to be recognized and measured (except of measurements of very thin samples when the relevant resonance is not much affected).
D) It should be mentioned, that Keysight (see: https://www.keysight.com/us/en/assets/7018-01496/technical-overviews/5989-6182.pdf) acknowledges, that samples of a specific combinations of permittivity and thickness cannot be measured in the SCRs due to coincidence of resonant frequencies of the desired and undesired modes. Application of the invention presented here practically eliminates that kind of restrictions.

The invention in general is applicable in axially symmetric structures which can be best described using three cylindrical components: radial component R, angular component Φ, and axial component - Z (typically later referred to as vertical). Axial symmetry allows description of the structure in two dimensions (R and Z) in the full range of the angular component 0< Φ <2π. In such a notation, a cylinder of radius R0 and height H can be described as a body of 0<R<R0 and 0<Z<H. A ring can be described as a body of R1<R<R2 and H1<Z<H2.

Person skilled in the art is capable of selecting and applying adequate coupling for the resonator to generate desired TEOnp modes.

The invention is in particular applicable for microwave resonators, composed of conductive walls and comprising a cavity of cylindrical shape. The cavity is filled predominantly by air, although it may contain inserts of dielectrics, typically Sapphire or ceramics, having the shape of cylinders or rings with the same axis of symmetry as the cavity. The shape of the cavity can deviate in form from that of a pure cylinder providing that its axial symmetry is substantially maintained. Small non-symmetrical perturbations such as coupling systems or small openings are not considered as affecting symmetry. Although embodiments discussed in the description are focused on SCR the invention is applicable in all resonating structures having a cavity substantially symmetrical with respect to an axis including in particular the ones discussed in the section concerning the state of the art.

## Claims

1. Electromagnetic resonating structure (**100,200,300,400**) comprising a substantially axially symmetrical cavity (**101**, **102, 401**) having an axis of symmetry (**Z**) and being at least partially surrounded by a wall (**111, 112, 411**) made of conducting material and adapted to support electromagnetic resonance at working mode having axially symmetrical all field components and E-field restricted to angular direction, **characterized in that** it is provided with at least one channel (**120, 220, 320, 420**) extending outwards from the cavity (**101**, **102, 401**) and connected with a volume of absorbing material (**130, 330, 430**), wherein the channel (**120, 220, 320, 420**) is configured to receive a field distributed according to at least one mode other than working mode and reflect back to the cavity field distributed according to the working mode.

2. Electromagnetic resonating structure according to claim 1, wherein the channel (**120, 220, 320, 420**) has a height within a range of 0.02 λ to 0.1 λ.

3. Electromagnetic resonating structure according to claim 1 or 2, wherein the channel (**120, 220, 320, 420**) has a length within a range of 0.2 λ to 0.7 λ.

4. Electromagnetic resonating structure according to claim 1 or 2 or 3, wherein the working mode is TE011.

5. Electromagnetic resonating structure according to any of claims 1-4, having more than one channel (**120, 220, 320, 420**).

6. Electromagnetic resonating structure according to any of claims 1-5 having at least one channel (**120, 320**) extending in a direction perpendicular to the axis of symmetry (**Z**) of the cavity (**101**, **102**).

7. Electromagnetic resonating structure according to claim 6 having a wall divided into a first wall (**111**) defining a first part (**101**) of the cavity and a second wall (**112**) defining a second part (**102**) of the cavity, the structure having at least two channels (**220**) each located in different part (**101**, **102**) of the cavity.

8. Electromagnetic resonating structure according to any of claims 1-4 having at least one channel (**220, 420**) extending in parallel to the axis of symmetry (**Z**) of the cavity (**101**, **102, 401**).

9. Structure according to any of claims 1-8 wherein the conducting wall (**111,112**) is divided into two parts by a plane perpendicular to the axis of symmetry (**Z**) of the cavity (**101**, **102**) leaving a slot adapted to receive a flat dielectric sample (**103**).

10. Structure according to any of claims 1-8 wherein the wall has open base perpendicular to the axis of symmetry (**Z**) of the cavity (**401**) and the open base is adapted to be applied to a flat sample under test.

11. Structure according to any of claims 1-10 having at least one axially symmetrical dielectric insert (**404**) with the axis of symmetry coinciding with the axis of symmetry (**Z**) the cavity, adapted for supporting a sample (**403**).

12. Structure according to claim 11 comprising two dielectric inserts (**404**) arranged symmetrically to the axis of symmetry (**Z**) and adapted for sandwiching the sample (**403**) between them.

13. Structure according to claim 11 or 12 wherein the insert comprises sapphire.

14. Structure according to any of claims 12-13, wherein the cavity is divided into
a first part (**401**) and second part and wherein each of the first part (**401**)
and the second part
each of the dielectric inserts (**404**) is located in a slot next to a cut separating the first part (**401**) and the second part and closes respective part, wherein
the wall of the cavity comprises additional channel (**421**) beginning next to the slot fitting the insert (**404**) and
the channel (421) comprises absorbing material (**431**) at its end.

15. A method of measuring of a material parameter of a sample under test, using a resonating structure comprising a cavity, the method comprising steps of
inducing a resonant mode of electromagnetic field, in the resonating structure without the sample under test and measuring a first response,
placing the sample under test in the resonating structure, inducing the same resonant mode of electromagnetic field in the resonating structure and measuring a second response,
determining the material parameter of the sample under test from the difference between the first and the second responses
**characterized in that**
the resonating structure is a resonating structure as defined in any of claims 1 to 14.
